# EUROPEAN PATENT SPECIFICATION

(11) **EP 2 736 600 B1**
(45) Date of publication and mention of the grant of the patent: **31.08.2016**
(21) Application number: 12773379.8
(22) Date of filing: 26.07.2012
(51) Int. Cl.: A61P 37/06, A61K 35/12, A61K 35/28

(54) **MICROVESICLES ISOLATED FROM MESENCHYMAL STEM CELLS FOR USE AS IMMUNOSUPPRESSIVE AGENTS**
AUS MESENCHYMSTAMMZELLEN ISOLIERTE MIKROVESIKEL ZUR VERWENDUNG ALS IMMUNOSUPPRESSIVA
MICROVÉSICULES ISOLÉES DE CELLULES SOUCHES MÉSENCHYMATEUSES DESTINÉES À ÊTRE UTILISÉES COMME AGENTS IMMUNOSUPPRESSEURS

(30) Priority: 28.07.2011 IT RM20110403
(43) Date of publication of application: 04.06.2014
(73) Proprietor: Cryo-Save AG, 8808 Pfäffikon (CH)
(72) Inventor: MURACA, Maurizio, I-00165 Roma (IT); FIERABRACCI, Alessandra, I-00165 Roma (IT)
(74) Representative: Gitto, Serena
(86) International application number: PCT/IT2012/000232
(87) International publication number: WO 2013/014691

(56) References cited:
- WO-A2-2006/007529
- CLOTILDE THÉRY ET AL: "Membrane vesicles as conveyors of immune responses", NATURE REVIEWS IMMUNOLOGY, vol. 9, no. 8, 1 August 2009 (2009-08-01), pages 581-593, XP055018445, ISSN: 1474-1733, DOI: 10.1038/nri2567
- VALADI H ET AL: "Exosome-mediated transfer of mRNAs and microRNAs is a novel mechanism of genetic exchange between cells", NATURE CELL BIOLOGY, NATURE PUBLISHING GROUP, GB, vol. 9, no. 6, 1 June 2007 (2007-06-01), pages 654-659, XP002546815, ISSN: 1465-7392, DOI: 10.1038/NCB1596 [retrieved on 2007-05-07]
- CAMUSSI GIOVANNI ET AL: "Exosomes/microvesicles as a mechanism of cell-to-cell communication.", KIDNEY INTERNATIONAL NOV 2010 LNKD- PUBMED:20703216, vol. 78, no. 9, November 2010 (2010-11), pages 838-848, XP002669206, ISSN: 1523-1755
- ESCUDIER BERNARD ET AL: "Vaccination of metastatic melanoma patients with autologous dendritic cell (DC) derived-exosomes: results of thefirst phase I clinical trial", JOURNAL OF TRANSLATIONAL MEDICINE, BIOMED CENTRAL, LONDON, GB, vol. 3, no. 1, 2 March 2005 (2005-03-02), page 10, XP021009856, ISSN: 1479-5876, DOI: 10.1186/1479-5876-3-10
- MORSE MICHAEL A ET AL: "A phase I study of dexosome immunotherapy in patients with advanced non-small cell lung cancer", JOURNAL OF TRANSLATIONAL MEDICINE, BIOMED CENTRAL, LONDON, GB, vol. 3, no. 1, 21 February 2005 (2005-02-21), page 9, XP021009899, ISSN: 1479-5876, DOI: 10.1186/1479-5876-3-9
- ELISABETTA TRAGGIAI ET AL: "Bone Marrow-Derived Mesenchymal Stem Cells Induce Both Polyclonal Expansion and Differentiation of B Cells Isolated from Healthy Donors and Systemic Lupus Erythematosus Patients", STEM CELLS, vol. 26, no. 2, 1 February 2008 (2008-02-01), pages 562-569, XP055018794, ISSN: 1066-5099, DOI: 10.1634/stemcells.2007-0528
- DATABASE MEDLINE [Online] US NATIONAL LIBRARY OF MEDICINE (NLM), BETHESDA, MD, US; November 2010 (2010-11), CAIMI PAOLO F ET AL: "Emerging therapeutic approaches for multipotent mesenchymal stromal cells.", XP002669207, Database accession no. NLM20729733 & CURRENT OPINION IN HEMATOLOGY NOV 2010 LNKD- PUBMED:20729733, vol. 17, no. 6, November 2010 (2010-11), pages 505-513, ISSN: 1531-7048
- I. RASMUSSON ET AL: "Mesenchymal Stem Cells Stimulate Antibody Secretion in Human B Cells", SCANDINAVIAN JOURNAL OF IMMUNOLOGY, vol. 65, no. 4, 1 April 2007 (2007-04-01), pages 336-343, XP055018762, ISSN: 0300-9475, DOI: 10.1111/j.1365-3083.2007.01905.x
- COMOLI PATRIZIA ET AL: "Human mesenchymal stem cells inhibit antibody production induced in vitro by allostimulation.", NEPHROLOGY, DIALYSIS, TRANSPLANTATION : OFFICIAL PUBLICATION OF THE EUROPEAN DIALYSIS AND TRANSPLANT ASSOCIATION - EUROPEAN RENAL ASSOCIATION APR 2008 LNKD- PUBMED:18029377, vol. 23, no. 4, April 2008 (2008-04), pages 1196-1202, XP002669208, ISSN: 1460-2385
- CORCIONE ANNA ET AL: "Human mesenchymal stem cells modulate B-cell functions.", BLOOD 1 JAN 2006 LNKD- PUBMED:16141348, vol. 107, no. 1, 1 January 2006 (2006-01-01), pages 367-372, XP002669209, ISSN: 0006-4971
- TABERA SORAYA ET AL: "The effect of mesenchymal stem cells on the viability, proliferation and differentiation of B-lymphocytes.", HAEMATOLOGICA SEP 2008 LNKD- PUBMED:18641017, vol. 93, no. 9, September 2008 (2008-09), pages 1301-1309, XP002669210, ISSN: 1592-8721
- LAMPARSKI H G ET AL: "Production and characterization of clinical grade exosomes derived from dendritic cells", JOURNAL OF IMMUNOLOGICAL METHODS, ELSEVIER SCIENCE PUBLISHERS B.V.,AMSTERDAM, NL, vol. 270, no. 2, 15 December 2002 (2002-12-15), pages 211-226, XP004387999, ISSN: 0022-1759
- S. Bruno ET AL: "Mesenchymal Stem Cell-Derived Microvesicles Protect Against Acute Tubular Injury", Journal of the American Society of Nephrology, vol. 20, no. 5, 1 May 2009 (2009-05-01), pages 1053-1067, XP055027861, ISSN: 1046-6673, DOI: 10.1681/ASN.2008070798
- HERRERA M B ET AL: "Human liver stem cell-derived microvesicles accelerate hepatic regeneration in hepatectomized rats", JOURNAL OF CELLULAR AND MOLECULAR MEDICINE, UNIVERSITY PRESS CAROL DAVILA, BUCHAREST, RO, vol. 14, no. 6B, 1 June 2010 (2010-06-01), pages 1605-1618, XP002730586, ISSN: 1582-1838, DOI: 10.1111/J.1582-4934.2009.00860.X [retrieved on 2009-07-24]

## Description

The present invention concerns microvesicles isolated from mesenchymal stem cells for use as immunosuppressive agents. In particular, the invention concerns microvesicles isolated from mesenchymal stem cells to be used as immunosuppressive agents for treatment of inflammatory and immune pathologies.

Immunomodulating properties of stromal mesenchymal stem cells are the object of growing interest and clinical application but the effect reproducibility thereof is controversial and involved mechanisms only partially have been elucidated. Mesenchymal stromal cells (MSC) displayed immunoregulating properties in vitro, in animal model and clinical applications as for graft-versus-host disease (see reviews by Nauta 2007, Zhao 2010, Caimi 2010). However, action mechanisms thereof are not completely elucidated. In particular, while MSC immunosuppressive effect on activated T lymphocytes more extensively has been studied (Krampera, 2003), and multiple involved factors described, both MSC effect on B lymphocytes and action mechanism are very controversial (Corcione 2006, Tabera 2008, Comoli 2008, Traggiai 2008, Rasmussen 2007). Being reported species differences (human vs. mouse) as to MSC and B cells interaction (Renseneb, 2009), below only results deriving from human cells will be discussed. Generally the occurrence of MSC-B lymphocytes interaction mediating soluble factors is assumed and indeed it has been also reported that these factors would have a more important effect on B than T lymphocytes (Augello, 2006).

As above reported, MSC effect on B lymphocytes does not seem to be univocal. In fact both inhibiting (Corcione 2006, Tabera 2008, Comoli 2008) and stimulating effects (Traggiai 2008, Rasmussen 2007) on antibody proliferation, differentiation and production by B lymphocytes have been described. Interestingly it is remarkable that in certain conditions, MSCs can play a role of antigen presenting cells (Chan, 2006), thus amplifying immune response. This phenomenon appears to occur under certain environmental conditions, for example in the presence of low gamma-interferon levels (Chan, 2006). According to a study on B lymphocyte interaction, MSCs displayed opposite effects (inhibiting or stimulating) depending on stimulus intensity used for B cells or donor origin (Rasmussen, 2007). These conflicting and not predictable results lead to impediments in MSC use for complex autoimmune disease treatment.

In the light of above it is therefore apparent the need to provide new treatments of inflammatory and immune diseases suitable to overcome the disadvantages of prior art.

Recently, it has been demonstrated that multiple interactions of immune cells are mediated by microvesicles (MV) or microparticles, i.e. membrane coated bodies secreted by many cell phenotypes (Théry, 2009). Various MV species have been described, sized from 50 to 1000 nm or more, and with different structural and biochemical properties depending on intracellular compartment deriving from. An increasing lot of experimental evidences suggest that said MVs play a fundamental role in intercellular communication, through protein, mRNA and microRNA transfer (Valadi, 2007; Camussi, 2010).

The role of MVs as immune response carrier has been demonstrated for cellular phenotypes like dendritic cells or T lymphocytes. Microvesicles isolated from dendritic cell expressing tumour antigen have been used in two phase I clinical trials on melanoma or pulmonary carcinoma suffering patients (Escudier, 2005; Morse, 2005), in order to stimulate tumour immune response. These studies demonstrated MVs to be safe for human systemic use, encouraging clinical result occurring for individual patients. The use of exosomes with immunosuppressive activity has been studied by P Robbins et al. (US20060116321) which isolated exosomes from different cell types including antigen presenting cells (dendritic cells and macrophages). Analogously the use of microvesicles from dendritic cells for immunotherapeutic purposes (Lamparsky, US 6812023) as prevention of not desired auto- or graft-antigen immune responses, or reduction of exaggerated antigen response (Mattews Albert L, US 20020004041) has been proposed.

Exosomes from antigen presenting cells as vaccine carriers (Delcayre A, US20060222654) or antitumor response generators (Delcayre A, US7704964) have been also studied.

Microvesicles derived from endothelial cells, preferably from endothelial progenitors, have been studied like possible adjuvants in transplant of islands endothelial pancreatic-cells for treatment of Type 1 or Type 2 diabetes. Adjuvanting effect would allow the survival and functionality of transplanted islands (Cantaluppi V, US20100233216).

Moreover it has been described that MSC inhibiting effect on B lymphocytes occurs also when said two cell populations are separated by permeable membranes, an observation that has been considered as a support of soluble mediators occurrence (Corcione, 2006; Augello, 2005). However, the inhibiting effect did not (Corcione, 2006) or only partially occur (Augello, 2005) when MSC culture supernatant was used. Therefore it has been assumed that MSC inhibiting effect in order to be completely exerted would need intercellular contact or B lymphocyte signal release (Corcione, 2006).

As to mesenchymal stem cells, microvesicles from mesenchymal stem cells (MSC-MVs) have been described as possible mediators of antiapoptotic and pro-regenerative effect associated to MSC administration in animal models (Bruno 2009; Herrera, 2009; Gatti, 2011).

The inventors of the present invention now have found that microvesicles isolated from stem cells are suitable to effectively mediate immunosuppressive effect on B lymphocytes. In fact, the inventors have identified specific mediators of inhibiting effect of mesenchymal stem cells on B lymphocyte in microparticles produced from mesenchymal stem cells, also called microvesicles (MV) and recently recognized like mediators of intercellular communication. Therefore, microvesicles or exosomes from mesenchymal stem cells can be advantageously used like immunosuppressive agents, in particular in chronic inflammatory pathology and autoimmune diseases as for example Type 1 diabetes. In particular, it has bees observed that using MVs deriving from mesenchymal stem cell culture medium a strong dose-dependent effect on antibody proliferation, differentiation and production by CpG stimulated B lymphocyte is obtained. Using a fluorescent label, it has been demonstrated both using FACS and confocal microscopy analyses that MVs are associated with CpG stimulated B lymphocyte but not with other immune cells like T lymphocytes, dendritic or NK cells, said result further supporting specificity of detected effect.

These results indicate that MVs produced by mesenchymal stem cells are carriers of inhibiting effect exerted by said cells on B lymphocytes. Therefore, MVs, produced by MSCs under certain culture conditions, can be therapeutically used instead of origin cells, resulting in remarkable advantages in terms of standardization, ease of handling, safety and effectiveness. Both using MSCs and MSC-MVs also functional inhibition of B lymphocyte is obtained, as demonstrated by reduction of immunoglobulin secretion. Moreover, a linear correlation of MSC-MV concentration and inhibition of B cell proliferation and differentiation is observed.

In order to verify whether observed phenomena were mediated by interaction with other cell populations occurring in PBMCs, MSC-MVs have been labelled using a fluorescent dye under above described experimental conditions and association with different cell phenotypes labelled with fluorescent antibodies against specific surface markers using both FACS and confocal microscopy analyses has been verified. MSC-MVs displayed to be associated exclusively with B and not T lymphocytes, NK or dendritic cells. These data indicate that the inhibiting effect of MSCs on B cells is greatly, if not completely, mediated by MV release.

Moreover, below reported experimental data show the *in vivo* anti-inflammatory effect of MSC-MVs. In particular, results obtained on animal model of ulcerous colitis show a clinical improvement and flogosis index reduction.

The use of MSC-MVs instead of whole living cells could therefore offer a series of meaningful advantages, both in terms of safety and ease of handling. The MSC-MV use moreover offers advantages both for the production and clinical use.

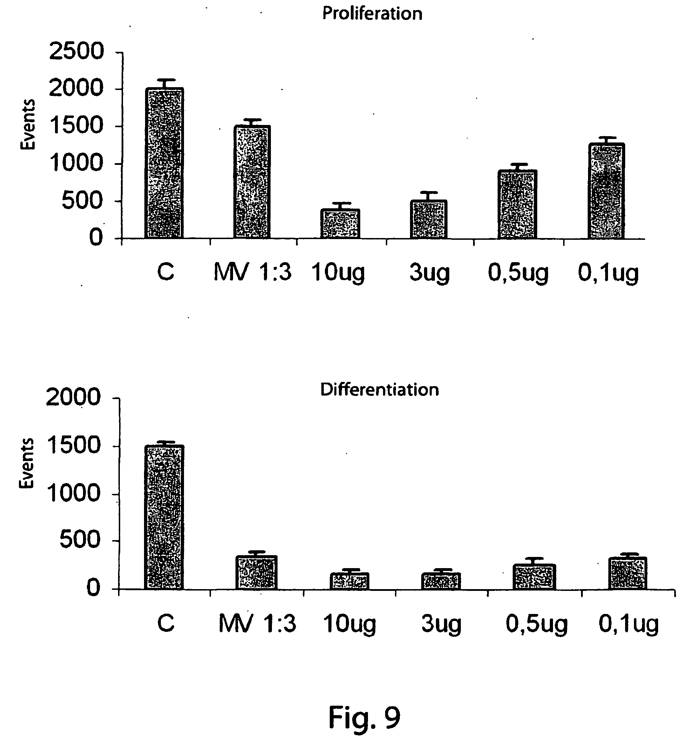

The advantages for the production are the following ones:
- MSC-MVs can be produced due to continuous release thereof by MSCs within the culture medium, wherefrom can be isolated by ultrafiltration/ultracentrifugation.
- The production thereof can be amplified using bioreactors allowing large cell amounts to adjusted within three-dimensional structures, and facilitate the isolation of continuous flowing MSC-MVs.
- The production of MSC-MVs by cells can be stimulated based on medium composition by addition of various substances (e.g. cytokine as gamma-interferon) or variable physical conditions (e.g. oxygen pressure).
- MSC-MVs can be isolated from medium by ultrafiltration, using filters with 30 to 100 kD cut-off.
- After the concentration by ultrafiltration, MSC-MVs can be purified by ultracentrifugation at 100000 g.
- Isolated and purified MSC-MVs can be optionally subjected to a sterilization process by irradiation, other than living cells.
- MSC-MV can be easy frozen in PBS, optionally with addition of cryoprotective agents approved for clinical use (DMSO), and after thawing washed by ultrafiltration/ultracentrifugation. Now the same are ready to be used, that is it is not necessary (other than cells) the verification of viability and functionality thereof.
- The obtained product is more easy standardizable than living cells; this results in higher compliance to pharmaceutical regulation and cost reduction.
- MSC-MVs can be used as carrier for effect amplifying molecules like annexin V (see figure 9)

As to clinical level there are the following advantages:
- MSC-MVs are quickly metabolized (Gatti, 2011) and do not generate ectopic colonization problems as living cells.
- MSC-MVs do not involve oncogenic transformation risk as it occurs using living cells, especially when in vitro manipulated (Tolar, 2007).
- MSC-MVs display a more specific and reproducible activity on activated target B cells (B lymphocyte) than MSCs.
- MSC-MV systemic administration, differently than MSCs, involves the infusion of very lower volumes of particle material resulting in lower embolization risk.
- MSC-MV repeated administration, also close to each other, do not involve above reported safety problems deriving from use of living cells, thus facilitating the attainment of desired effect.
- MSC-MVs are simpler and more stable bodies than origin cells and display a reproducible and expectable behaviour, on the contrary MSC fate after transplant is uncertain. For example, MSC behaviour can vary depending on interaction environment, as also confirmed by our experimental data. On the contrary environmental condition did not effect MSC-MV activity on lymphocytes.
- All the above reported advantages assume still greater importance at paediatric level, due both to ethical reasons and smaller body sizes and longer life expectancy.

It is therefore a specific object of the present invention microvesicles, or exosomes, isolated from mesenchymal stem cells for use in immunosuppressive therapy.

The term microvesicles refers to microparticle mixture having sizes generally variable about 0.1 to 1 micron.

In particular, the microvesicles according to the invention can be used for treatment of inflammatory, inflammatory chronic and autoimmune diseases. Inflammatory and autoimmune pathologies, in particular antibody-mediated diseases, can be severe and chronic. Treatment with microvesicles of the invention aims to reduce flogosis extent, symptoms and correlated complications. Chronic inflammatory diseases are for example autoimmune inflammatory diseases, rheumatology diseases like inflammation of connective and vascular tissues. For example, diseases that can be treated with MVs according to the present invention are the following: systemic vasculitis, polyarteritis nodosa, giant cell arteritis, Wegener's granulomatosis, Henoch-Schonlein purpura, cryoglobulinaemia, central nervous system vasculitis, multiplex mononeuritis, Takayasu's arteritis, Behçet's disease, Buerger's disease, intestinal chronic inflammatory diseases, as for example Crohn's disease and ulcerative colitis, Hashimoto's thyroiditis, autoimmune hemolytic anemia, Addison's disease, diabetes mellitus Type I, adult late-onset autoimmune diabetes (LADA), recurrent autoimmune diabetes in longstanding diabetes patients after receiving pancreas or islet transplantation, rheumatoid arthritis, systemic lupus erythematosus, dermatomyositis, scleroderma, Sjogren's syndrome, multiple sclerosis, chronic autoimmune hepatitis, primary biliary cirrhosis, psoriasis, alopecia areata, vitiligo, Goodpasture's syndrome, Guillame-Barrè's syndrome, chronic glomerulonephritis, dermatitis and eczema, Reiter's syndrome, reactive arthritis, cystic fibrosis, sinusitis, chronic bronchitis, periodontal disease, diverticulosis/diverticulitis.

Moreover the microvesicles according to the present invention can be used for treatment and prevention of transplant rejection. For example said microvesicles can be used in transplants of cells, tissues and solid organs, aiming to favour donor/acceptor compatibility, both reducing immune response and favouring development of immunity tolerance: graft-versus-host-disease, transplant rejection (graft) both for cell and solid organ. The microvesicles of the invention can be further used in order to inhibit immune reaction developing as result of allogeneic/xenogeneic cell transplant or gene therapy.

It is a further object of the present invention a pharmaceutical composition comprising or consisting of microvesicles isolated from mesenchymal stem cells, as active principle, optionally binding molecules enhancing immunosuppressive activity thereof as for example annexin, together with one or more pharmaceutically acceptable excipients and/or adjuvants for use as described in claims 6-10.

The present invention now will be described by an illustrative but not limitative way according to preferred embodiments thereof with particular reference to enclosed drawings wherein:

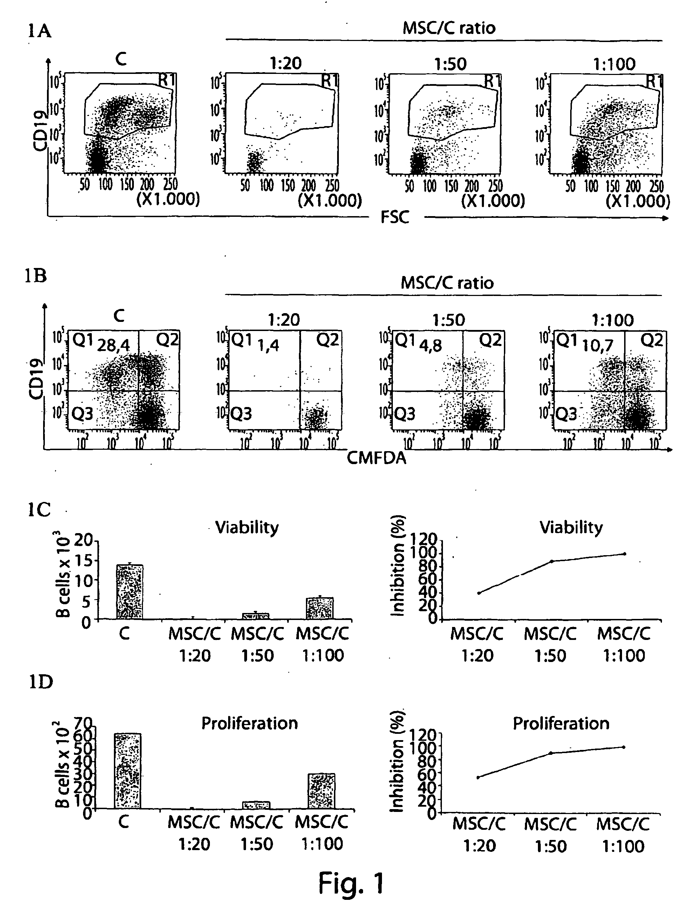

Figure 1 shows a co-culture of CPG and MSC stimulated CLMs. 1A-1B) Density plots representative of cytofluorimetric analysis of CFMDA labelled CLMs (c) or CLMs after co-culture with SCMs (MSC/C) at 1:20, 1:50 and 1:100 MSC/C ratio. 1A) CD19 positive cells were selected at R1 gate from only CLM culture (C) or MSC co-culture. 1B) In the upper left quadrant Q1 the percentage of CD19/CMFDA positive cells is shown for only CLM culture (C) or MSC co-culture. 1C) Histogram (left) and plot (right) showing the mean and inhibition percentage, respectively, of CD19 positive lymphocyte viability (B cells) for 3 independent experiments from only CLM culture (c) or MSC co-culture. 1D) Histograms (left) and plot (right) showing the mean and percentage of proliferation inhibition, respectively, of CD19 positive and CMFDA labelled cells for 3 independent experiments from only CLM culture (c) or MSC co-culture.

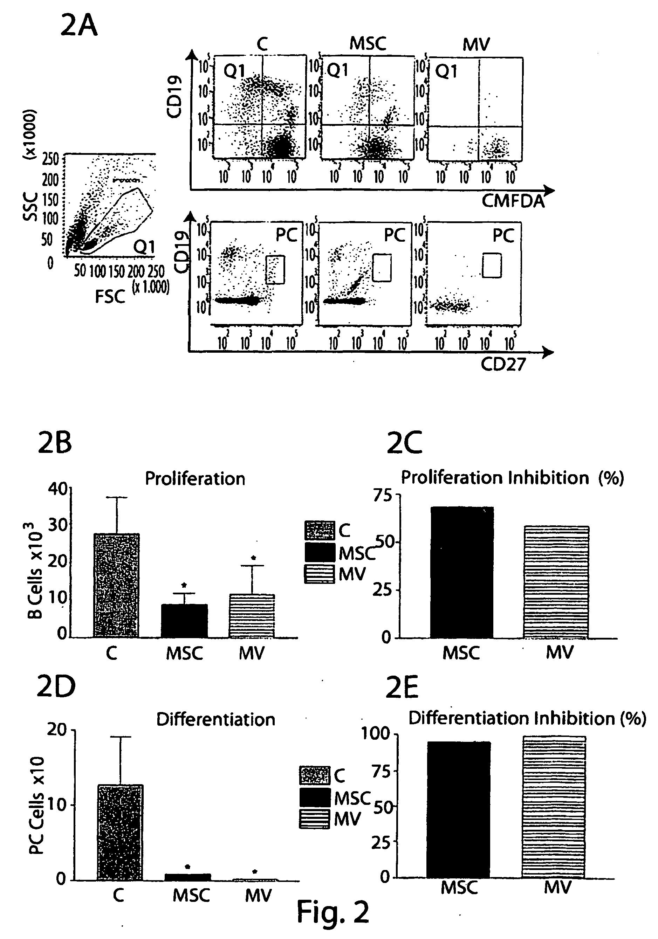

Figure 2 shows a parallel co-culture of CpG, MSC or MSC-MV stimulated CLMs. 2A) Density plots representative of cytofluorimetric analysis of CMFDA labelled CLMs of control group (C) and CLMs after co-culture with MSC or MSC-MV. Lymphocytes were selected at Q1 gate by morphologic parameters (FSC-H; SSC-H) like viable cells (left). Said lymphocytes are successively analyzed based on proliferation rate by selection of CD19/CD27 positive events (right, upper panel) or by differentiation phase in plasma cells (PC) by selection of CD19/CD27 positive events (right, lower panel). 2B-2C) Histograms showing the mean and proliferation inhibition percentage, respectively, of CMFDA/CD19 positive lymphocytes from only CLM culture (C) or MSC or MSC-MV co-culture. 2D-2E) Histograms showing the mean and percentage of proliferation inhibition, respectively, of CD19/CD27 positive plasma cells (PC) from only CLM culture (C) or MSC or MSC-MV co-culture. The results are mean and standard error of 3 independent experiments. p<0.05

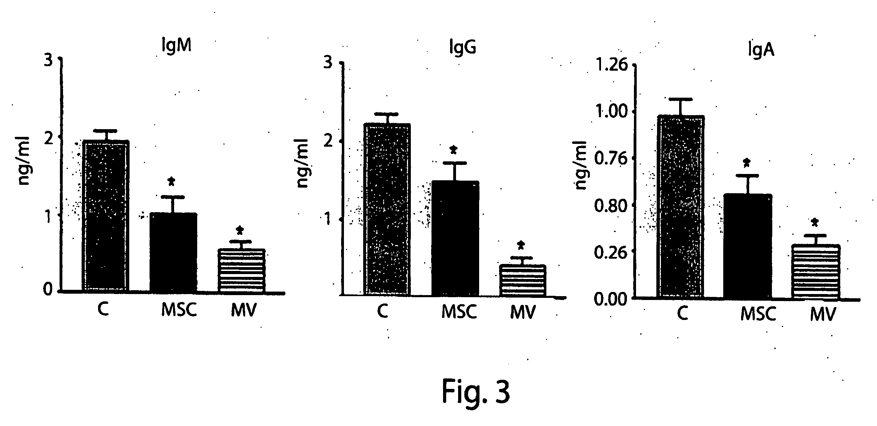

Figure 3 shows the inhibition of immunoglobulin production in CpG stimulated CLMs after MSC or MSC-MV co-culture. ELISA assay of supernatants from only CLM culture (C), incubated with MSC or MSC-MV. The results are mean and standard deviation of IgM, IgG or IgA concentration (ng/ml) for 3 independent experiments. *p< 0.05

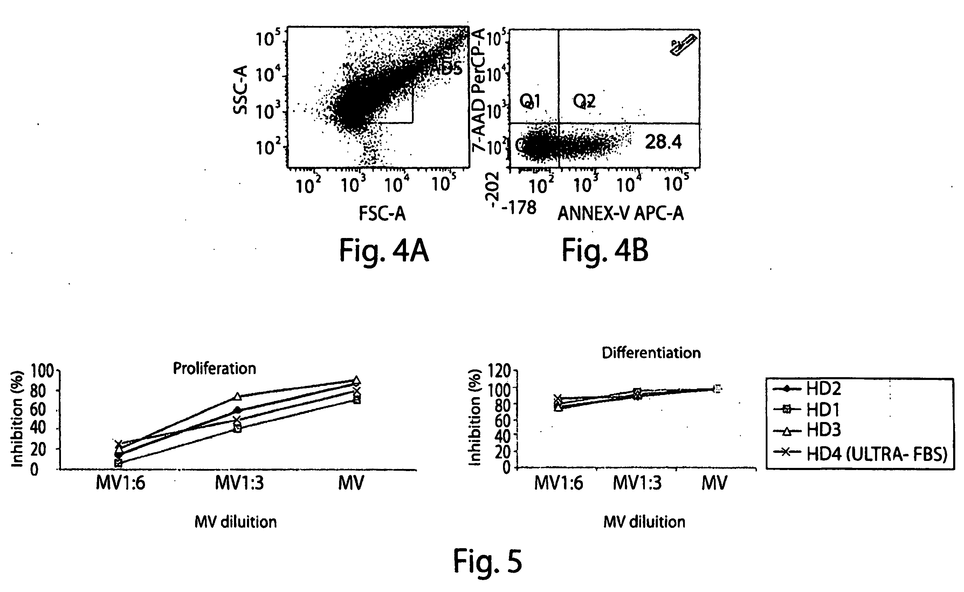

Figure 4 shows the characterization of MSC-MVs. Representative scatter plot obtained for MSC released MVs using FACSCanto cytofluorimeter. 4A) MV gate was selected using 0.5-1µm size calibration beads. 4B) MSC-MVs derived from gate shown in 4A were identified in Q4 quadrant by annexin V,7-AAD and Trucount (P1) beads.
Figure 5 shows the dose-dependent effect of MSC-MVs on B lymphocytes in CLMs. Panel A shows the inhibition of proliferation (left) and differentiation (right) of CpG stimulated CLMs after MSC-MV culture not diluted (MV) or at 1:3 or 1:6 dilution. A strong inhibition is observed. Results are from 4 independent experiments.

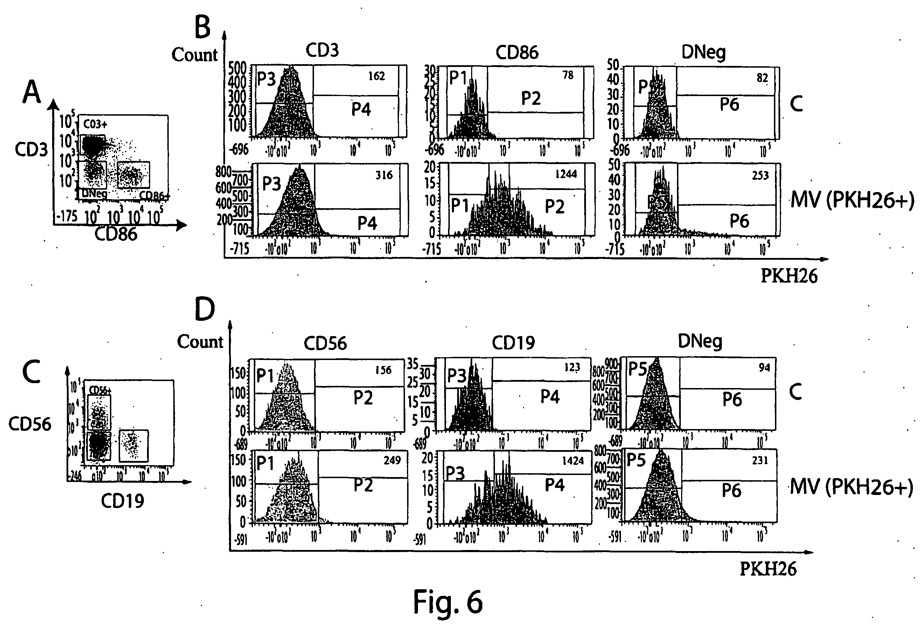

Figure 6 shows FAC analysis for incorporation of PKH26 labelled MVs by CLM selected populations after CpG stimulation. 6A) Density plots for CD3+, CD86+ lymphocytes and double negatives (Dneg) selected in 3 different gates; 6B) Histogram plots of fluorescence intensities for CD3-FITC, CD86-APC positive lymphocytes selected and analyzed before (upper panel) or after incubation with PKH26 labelled MSC-MVs (lower panel). Fluorescence intensity is higher in CD86 positive cells. 6C) Density plots for CD56+, CD19+ lymphocytes and double negatives (Dneg) selected in 3 different gates; 6B) Histogram plots of fluorescence intensities for CD56-FITC, CD19-APC-Cy7 positive lymphocytes selected and analyzed before (upper panel) or after incubation with PKH26 labelled MSC-MVs (lower panel). Fluorescence intensity is higher in CD86 positive cells.

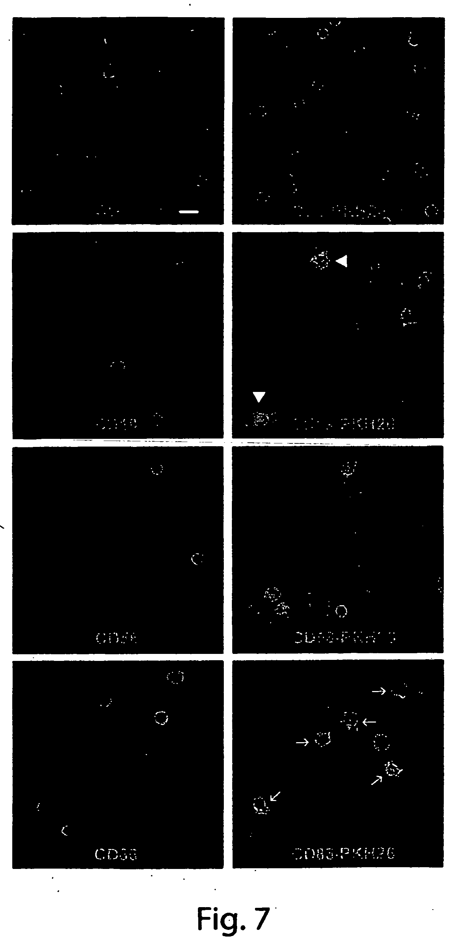

Figure 7 shows CLM confocal microscopy analysis. Cells have been stained with APC conjugated antibodies against CD3, CD19 and CD86 (green pseudo staining) and conjugated anti-CD 56 (green) with addition (upper panel) or without (left panel) incubation with PKH26 stained MSC-MV (red). MVs mostly occur within cytoplasm of CD19 (arrow tip) and CD86 (arrow) positive cells. Nuclei were counterstained with Hoechst. Magnification bar: 10µm.

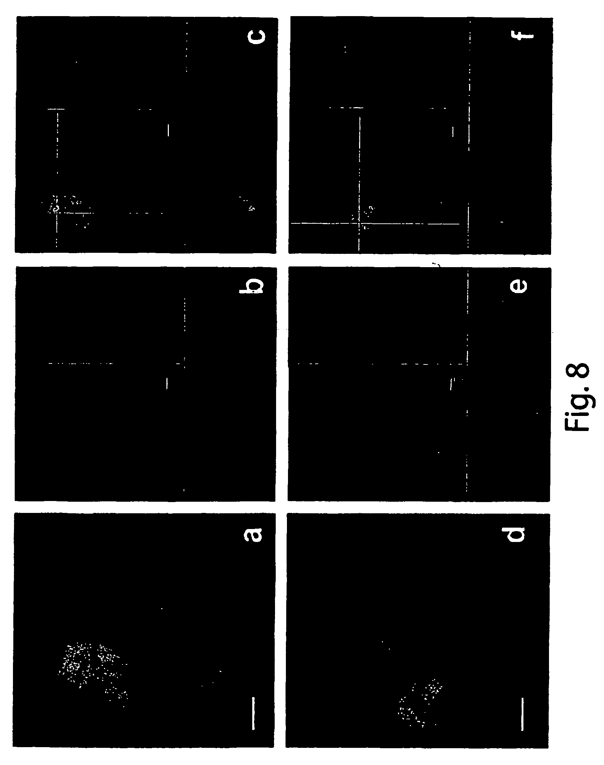

Figure 8 show Z reconstruction confocal microscopy for cell subpopulations. Z reconstruction microphotographies (a, d) carried out using laser scanning confocal microscopy of PKH26 positive MSC-MV incubated lymphocytes, stained with anti-CD19 (a-c) and anti-CD86 (d-f) antibodies. The projections on XZ and XY (b-c, e-f) axes obtained from consecutive multiple optical cross-sections showed intracellular localization of fluorescent red dye labelled MSC-MVs in CD19 positive (c, green) and CD86 positive (f, green) cells, respectively. Nuclei are stained using Hoechst. Magnification Bars: 5 µm (a, d) and 2 µm (b-c, e-f).
Figure 9 shows the histograms showing mean of proliferating and differentiating cells obtained from a single PBMC culture (c), with MV diluted 1:3 or MV 1:3 incubated with annexin V (concentration from 10 µg to 0.1 µg). The inhibiting effect of MVs is enhanced by annexin V according to dose-dependent mode.

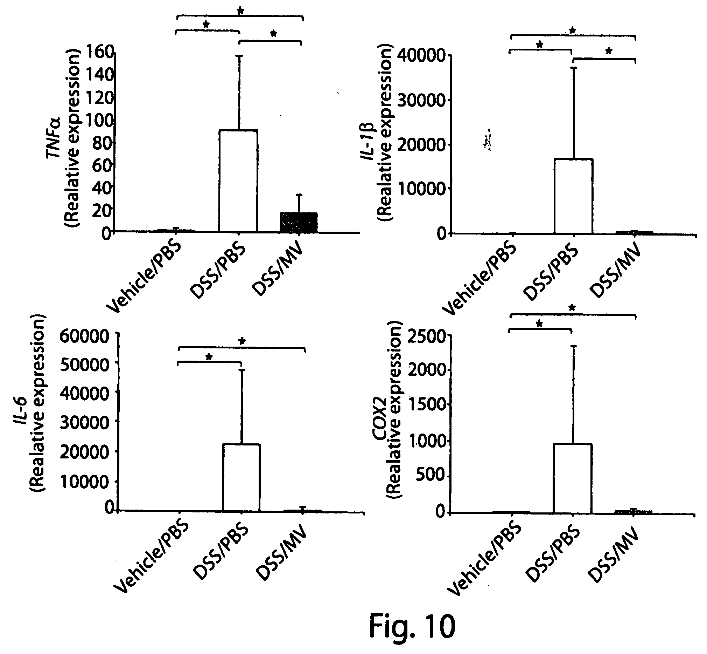

Figure 10 shows the expression of TNF alpha, IL-1beta, IL-6 and Cox2 in colon tissue on sixth day of experimentation in control animals without colitis induction administered only with PBS (vehicle/PBS), animals with DSS induced colitis and only PBS administered (DSS/PBS) and animals with DSS induced colitis and administered with injections of microvesicles isolated from stem mesenchymal cells and suspended in PBS (DSS/MV). Values are mean ±SD. *p<0.05.

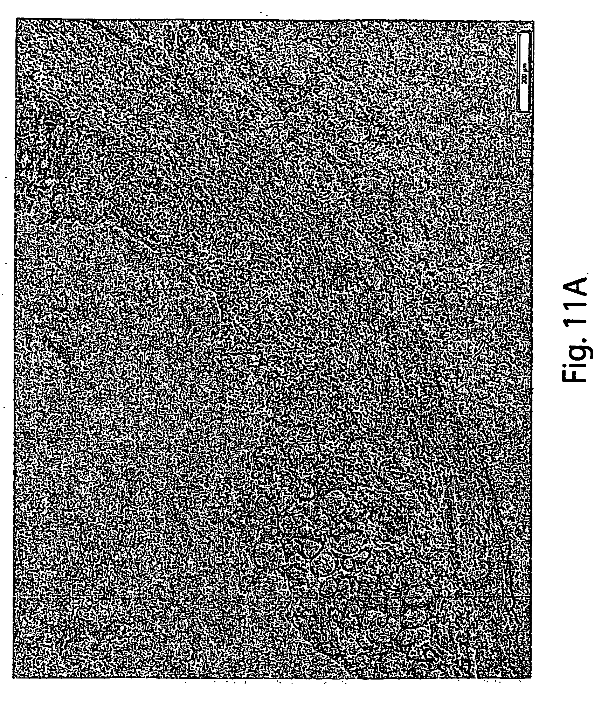

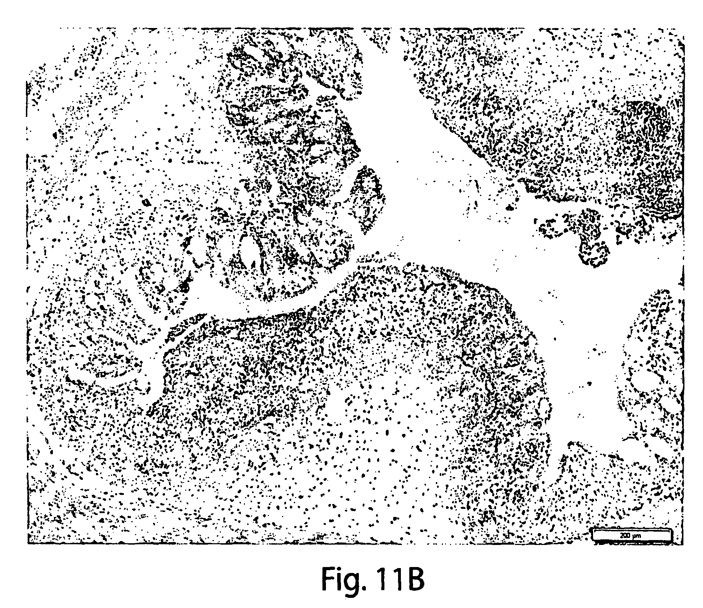

Figure 11 shows histological analysis (hematoxylin-eosin) of colon tissue from mouse with DSS induced colitis and administered with only PBS (A) and mouse with DSS induced colitis and administered with injections of microvesicles isolated from stem mesenchymal cells (b). Within mucosa and submucosa of treated mouse an inflammatory infiltrate with marked limphomononuclear and histiocyte component is visible.

### Example 1: Isolation of microvesicles from mesenchymal stem cells and in vitro study on effect of microvesicles on B lymphocytes

### MATERIALS AND METHODS

### Culture and expansion of mesenchymal stem cells

Marrow derived commercial human mesenchymal stem cells (MSC; Lonza, Basel, Switzerland) have been plated in 75cm² polystyrene culture flasks with ventilated stopper (Becton Dickinson, USA) at density of 4X10³ cells/cm³ in 10ml of Mesencult basal culture medium (Stem Cell Technologies, BC, Vancouver, Canada) added with bovine foetal serum (20%, FBS, Hyclone Laboratories), penicillin at 100 U/ml and streptomycin at 100 µg/ml (Gibco, Grand Island, NY). Cultures were incubated at 37°C in humidified atmosphere at 5% of CO₂. Afterwards cells were maintained within same culture medium, plate detached in trypsin/EDTA solution (Invitrogen, Life Technologies, Italy) at 80-90% confluence and re-plated at 1:2 dilution. Culture medium replacement occurring weekly.

### Isolation of limphomononuclear cells from peripheral blood

Blood samples from healthy donors are collected at Centro Trasfusionale Ospedale Pediatrico Bambino Gesù in Rome. After informed consent acquisition, cells peripheral blood limphomononuclear (CLM) have been separated by Ficoll gradient (Histopaque, Sigma Chemical C., St Louis, MO, USA) using 50 ml of heparinized venous blood, washed two times in saline phosphate buffer, then cryo-stored in liquid nitrogen.

### Co-Culture of limphomononuclear cells from peripheral blood with MSC

Experiments have been set up in order to test B lymphocyte viability in co-culture with adhered MSCs in 96 well culture plates (Corning-Costar, Celbio, Milan, Italy) at initial concentrations of 2.5x10⁴, 1X10⁴ or 5x10⁴ cells/well in 20% FBS added basal Mesencult culture medium. For immunomodulation tests MSCs are adhered to 96 well culture plates at lower concentration of 5x10³ cells/well and cultured in 20% FBS added basal Mesencult culture medium. On next day in MSC adhered wells, culture medium is drawn and replaced with CLMs (5x10⁵/well corresponding to 1:20, 1:50, 1:100 MSC/CLM ratio, respectively). MSCs have been previously labelled with 5-chloromethylfluorescein diacetate (CMFDA, Cell Tracker, Molecular Probes) at 0.5µM concentration and co-cultured with MSC in 10% FBS added RPMI 1640 culture medium (BioWhittaker, Lona, Belgium). B lymphocyte have been stimulated by incubation with 2.5µg/ml of CpG human oligodeoxynucleotides (Hycult Biotechnology). After one week cultured cells were washed in Dulbecco saline phosphate buffer (D-PBS, Euro-Clone, Milan, Italy) and analyzed using FACS (Fluorescent Activating Cell Sorter Analysis, FACSCanto II, BD Biosciences).

### CLM Co-Culture with MSC derived microvesicles (MSC-MV)

90 % confluent MSC cultures were used for preparation of conditioned medium (CM). After 5 day culture, medium is recovered from plates wherein cell plating has been initially carried out at 2x10⁶/well concentration and centrifuged at 1000 g for 20min in order to remove cell debris. MSC produced microvesicles (MSC-MVs) then have been isolated as described in successive paragraph, and added to 5x10⁵ CFMDA labelled lymphocytes after 1 hour and 24 hours of CpG stimulation (Hycult Biotechnology). For selected tests, MSC-MVs were diluted at 1:3, 1:6 in RPMI 1640 medium (BioWhittaker), then added to CLM labelled cultures after 1 hour and 24 hours from culture starting. After a week in the presence of 2.5 µg/ml of CpG human oligodeoxynucleotides added at culture starting, CLMs were recovered by centrifugation, washed and FACS analyzed.

### Detail of isolation methodology of microvesicles from culture medium

Isolation methodology of microvesicles is a Lamparski et al (2002) modified method
1 90% confluent MSCs: n=6 T75/cm² (11 ml/plate)
2 recovery of culture medium
3 centrifuge at 1000g for 10 min in order to eliminate cellular debris
4 recover 10ml of medium from each sample
5 concentrate medium using 4 ml 30kDa filters (Millipore)
6 recover concentrated medium (approximately 50µl for each filter) and dilute in PBS 10ml in ultra-centrifuge tubes
7 ultracentrifuge at 100000 g for 1 h at 4°C
8 recover MSC-MV pellet and dilute in PBS 10ml
9 concentrate MSC-MV solution using 4 ml 30kDa filters (Millipore)
10 recover approximately 15-20µl of selected sample (MSC-MV)

For isolation of microvesicles from MSCs, conditioned medium obtained according to above described procedure is diluted in 10ml of PBS in polyallomer tubes (Beckman Coulter, Milan, Italy), then ultracentrifuged at 100000 g at 4°C for 1 hour. At the end of procedure, 2 ml of ultracentrifuged medium were collected from bottom of the tubes, diluted in 10ml of PBS and concentrated by centrifugation for 20-30min at 2000 g in 30kDa MWCO Amicon Ultra Centrifugal sterile filter (Millipore) to 15-20µl final volume.

### Evaluation of immunoglobulin production

CFMDA labelled CLMs were co-cultured in 96 well plates both with MSCs (at 1:100 ratio) or MSC-MVs in FBS 10% (Hyclone) added RPMI 1640 (BioWhittaker) medium. After a week the plates were centrifuged at 300 g for 5 min, the supernatants recovered and tested using ELISA assay (enzyme-linked immunosorbent assay). In order to estimate the immunoglobulin production, goat purified immunoglobulin against PBS diluted human IgA, IgG or IgM (IgA 10µg/ml, IgG 15 µg/ml, IgM 2,5 µg/ml, Jackson ImmunoResearch Laboratories, West Grove, PA), were adhered at surface of ELISA 96 well plates (Corning) by overnight incubation at 4°C. After 3 0,1% PBS/Tween washings cell culture supernatant was added to plates (50µl/well) and incubated at 37°C for 1 hour under humidified atmosphere. After washing, the plates were incubated for 1 hour at 37°C with peroxidase conjugated antibodies against human IgA (1:1000), human IgG (1:2000) or human IgM (1:1000) diluted in PBS (Jackson ImmunoResearch Laboratories) (50 µl). PBS diluted o-phenylendiamine tablets (Sigma-Aldrich) were used as chromogen substrate in order the assay to be carried out. The reaction was interrupted after 30 min by addition of 10% sodium dodecylsulfate (50µl/well). At the end of the procedure plate readings were carried out using spectrophotometer (microplate spectrophotometer Benchmark Plus) at 460 OD.

### Cytofluorimetric Analysis

At co-culture end CLMs were recovered from culture plates, centrifuged at 300 g for 5 min and resuspended in PBS/FBS (2%). Single cell suspensions were incubated under dark for 20 min at 4°C with directly conjugated monoclonal antibodies against following surface human molecules: CD19 (1:7, Cy5 conjugated), CD27 [1:7, phycoerithrin (PE) conjugated], CD38 (1:30, PE-Cy7 conjugated), IgM (1:100, Cy5 conjugated), CD86 (1:5, APC conjugated), CD3 [1:7, fluorescein (FITC) conjugated], CD56 (1:7, FITC conjugated), CD19 [1:20 allophycocyanin-Cy7 (APC) conjugated]. All the antibodies were from Becton Dickinson (BD, Franklin Lakes, NJ, USA). After antibody binding, cells were washed 2 times with 2% PBS/BSA and data acquired using FACSCanto II (BD). Cytofluorimetric profiles were analyzed by Cell Quest Software (BD). A minimum of 20,000 events was recorded for single assay.

In order to characterize MSC-MVs, in individual assays, MSC-MVs from supernatants of 2x10⁶ MSCs according to above reported method were incubated with 5µl of monoclonal antibody against human surface molecule of APC conjugated annexin V (BD) complexed with vital 7-amino-actinomycin dye [7-AAD, conjugated with peridinin-chlorophyll-protein (PerCP) BD complex] in 500µl final volume of annexin V 1 x binding buffer (BB1 x) according to kit reported instructions. This complex allows specific identification of MSC derived not apoptotic MSC-MV. After 15min of incubation under dark at room temp, MSC-MV samples were transferred in Troucount (BD) tubes containing beads for dimensional calibration. Said beads were used in order to define MSC-MV gate based on morphologic parameters (forward scatter, FSC-H; side scatter, SSC-H).

### MSC-MV Incorporation by CLM

MVs are isolated from culture medium of 2X10⁶ MSCs *(see above)* and labelled with PKH26 dye at 2.5x10⁻⁶ (Sigma, Saint Louis, USA) concentration according to producer instructions. Labelled MVs were incubated with CLM (5X10⁵ cells/well) in 10% FBS added RPMI. After 1 hour of incubation at 37°C under humidified atmosphere with 5% of CO₂, the cell population was washed with PBS and divided in 2 parts, one assayed by cytofluorimetry, the other by confocal microscopy.

### Immunofluorescence

CLMs incubated with PKH26 labelled MVs were washed with PBS, fixed in 4 % formaldehyde, incubated with 5% PBS/FBS for 30 min and individually stained with following monoclonal antibodies: anti-CD86 (1: 10), anti CD3 (1:10) and anti-CD19 (1:10) conjugated with APC (BD) and FITC conjugated (1:30) anti-CD56 (BD). All the antibodies were diluted in 1% PBS/BSA and incubated for 1 hour. Negative controls were prepared using CLM samples without MV incubation, or without primary antibody in staining procedure.

Nuclei were counterstained with Hoechst 33342 1µg/ml (Invitrogen, Molecular Probes, OR, USA). After washing with PBS, samples were mounted with Pro-long Anti-Fade (Invitrogen) reagent.

### Confocal Microscopy and image analysis

Confocal analysis has been carried out using Olympus Fluoview FV1000 confocal microscope including FV10-ASW version 2.0, Multi Ar (458-488 and 515nm) software with diode laser 2X He/Ne (543 and 633 nm) and 405 using 60x (1,35 NA oil) lens. Individual optical sections are acquired by 1024X1024 pixel scanning mode, with 207 nm/pixel size, at 40 µsec/pixel sampling speed and 12 bit/pixel images. Fluorochrome mixing was avoided by automatic sequential acquisition of multichannel images, in order to reduce channel spectrum interference. 'Pinhole' opening was 1 Air unit.

Z reconstruction of single serial optical sections was carried out according to 1024x1024 pixel scanning mode with 2 electronic zoom corresponding to 103 nm/pixel, with 20 µs/pixel sampling speed and Z separation of 0.40 µsec/section. Images were processed using Photoshop 9,0 version software (Adobe System Inc, CA).

### Statistical Analysis

Data are analyzed in terms of mean, maximum and minimum quantitative value of data. Non parametric analysis of variance (Kruskal-Wallis test) is carried out in order to compare group quantitative data; Mann-Whitney U test has been used like test after variance analysis. P<0.05 was considered statistically significant. Statistical analysis was carried out using GraphPad software (GraphPad Prism, Release 3.03, Avenida de la Playa La Jolla, CA)..

### RESULTS

### CLM co-culture with MSC

### Effect on B cell viability

Firstly we have studied the effects of co-culture with MSC on viability of B lymphocytes after stimulation with CpG. We have co-cultured with MSCs CMFDA labelled CLMs (C) obtained from 7 healthy donors with MSC. After 7 day incubation we have analyzed by cytofluorimetric analysis CD19 positive cells in total sample of labelled CLMs. Maximum inhibition of B cell viability was observed at MSC/C 1:20 ratio (Figure 1 A); this effect was still provable 1:50 and 1:100 dilutions (Figure 1A, 1B).

### Effect on B cell proliferation

We have further estimated the effect of MSC co-culture on B cell proliferation. After 7 days under above reported experimental conditions the gate of double CD19/CMFDA labelled was selected by cytofluorimetric analysis. As it is apparent in Figure 1C we obtained maximum inhibition on B cell proliferation at MSC/C 1:20 ratio. In addition inhibition at MSC/C ratio of 1:50 and a lower effect at 1:100 ratio were provable. Q1 quadrant of Figure 1D shows the percentage of proliferating B lymphocytes (CD19 positive and CFMDA labelled) with respect to lymphocyte total number; the proliferation was already highly inhibited (90,3%) at MSC/C 1:100 ratio. Therefore, this ratio is used in all the successive co-culture experiments.

### CLM parallel co-culture with MSCs or MSC-MVs

In parallel experiments we have studied the effects of co-culture with MSCs or MSC-MVs on B lymphocyte proliferation and differentiation. To this end MSC-MVs are added to CLMs after 1 hour and 24 hours from the beginning of experiment. Parallel cultures wherein CLMs were co-cultured with MSCs at same 1:100 ratio were prepared. A meaningful inhibition on B cell proliferation is observed both in the presence of MSCs and MSC-MVs (Figure 2A). Similar inhibition percentages, i.e. 70% and 60%, respectively, were obtained both in the presence of MSCs and MSC-MVs (Figure 2B). In these parallel experiments we have also studied plasma cellular differentiation. Plasma cellular line (PC) was identified by double staining with CD19 and CD27. We have demonstrated a meaningful inhibition of plasma cellular production, being 98% and 100%, respectively, in the presence of both MSCs and MSC-MVs. As MSCs, MSC-MVs also inhibited the production of IgM, IgG and IgA (Figure 3).

Moreover we have estimated the production of immunoglobulins from same cell cultures. After 7 days, supernatants were recovered and assayed by ELISA. Both MSCs and MSC-MVs inhibited the production of IgM, IgG and IgA as shown in Figure 3.

### Characterization of MSC-MVs

We have verified that our method allowed purified MSC-MVs to be isolated, estimating also amounts by cytofluorimetric analysis. MSC-MV population is distinguished from fragments of apoptotic cells by DNA 7-AAD marker negativity and displayed to be annexin V positive (Figure 4).

### Dose-dependent effect of MSC-MVs on B lymphocytes

A dose-dependent effect of MSC-MVs on both B lymphocyte proliferation and differentiation has been displayed (Figure 5). The inhibition effect was greater on differentiation than proliferation.

### MSC-MV incorporation by CLMs

Cytofluorimetric analysis of CLMs pre-incubated with PKH26 labelled MSC-MVs demonstrated that incorporation thereof was selective in a sub-population of CD86 positive cells and among these in CD19 positive cells corresponding to B lymphocytes. Lymphocyte T corresponding CD3 positive cells displayed to be negative. NK cell corresponding CD56 positive cells displayed to be negative also (Figure 6).

Using laser confocal microscopy, immunofluorescence analysis of CLMs pre-incubated or not with PKH26 labelled MSC-MVs was carried out, in order to estimate the interaction of MSC-MVs with specific cell type (Figure 7). Panel of fluorescein conjugated antibodies against CD3, CD19, CD56 and CD86 molecules was used and co-localization thereof demonstrated a recurrent association of MSC-MVs with CD19 and CD86 positive cells, while no association was detected for CD19 and CD56 positive cells (Figure 7). Localization analysis of PKH26 labelled red stained MSC-MVs was based on Z reconstruction of single serial optical sections of CD19 and CD86 positive cells (Figure 8). MSC-MV distribution in lateral and axial dimensions, detected using XZ- and YZ axis projections, suggested an intracellular localization in the cytoplasm of CD19 and CD86 positive cells (Figure 8).

### EXAMPLE 2: MVs binding to annexin V enhances the inhibiting effect on B lymphocytes

Annexin V binds cell membrane exposed phosphatidylserine and modifies immune response against said cells (Munoz, 2007). In this experiment, MVs obtained according to above described procedure and 1:3 diluted are incubated for 15min with recombinant purified annexin V at room temp. (BD, concentration range from 10 µg to 0.1 µg) in 100 µl final volume of annexin V binding buffer (BBB1 x) according to producer instructions. Then MVs have been washed with PBS by ultracentrifugation and co-cultured with CFMDA labelled and PBMC stimulated CpGs. After 7 days cultures are analyzed by cytofluorimetry in order to estimate B lymphocyte proliferation and differentiation. Addition of annexin V remarkably enhanced MV inhibiting effect on activated B lymphocytes, in dose-dependent mode. This strategy can be used in order to enhance MV immunomodulating effect.

### EXAMPLE 3: Treatment of experimental ulcerous colitis with microvesicles derived from mesenchymal stem stromal cells (MSC-MVS)

This test is in order to estimate MSC-MV inhibiting effect for therapeutic purpose in experimental model of internal chronic inflammatory disease.

### Animal Model

Experiment has been carried out on female 8 week age B57BL/6J mice. Colitis has been induced by administration of 3% sodium dodecyl sulfate (DSS) in drinking water, *ad libitum* administered over five days. This is a well established and widely used animal model, being referenced hundreds times by Pub Med (for a review, see Kawada et al., 2007). All the animals have been treated according to the ethical code of L'Aquila University, wherein experiments have been carried out, having *ad libitum* availability of water and food and kept under 12 hour dark/light cycle. Animals are subdivided in three experimental groups.
- Group 1 (n=4, normal controls): intraperitoneal (ip) daily injections of PBS (only vehicle), 0,2 ml from 1 to 5 day;
- Group 2 (n=5, colitis induction): like group 1, with addition of 3% DSS in drinking water.
- Group 3 (n=4, colitis induction and treatment with microvesicles): like group 2, with addition of MSC-MVs suspended in 0.2 ml PBS by IP route.

On sixth day, the animals were sacrificed using CO₂. Colon has been excised, a part of tissue fixed in formalin for successive histological analysis, another part immediately frozen in liquid nitrogen and then stored at -80°C for successive RNA extraction.

### Dosage and administration route of MSC-MVs

MSC-MVs produced from 2x10⁶ MSCs over 24 hour culture have been isolated and administered according to previously described procedure.
Administration route: MSC-MV suspended in 0.2 ml of PBS by intraperitoneal way daily administered from 0 to 5 day.

### Evaluation of intestinal damage

### Animal weight

Disease activity index (faeces evaluation; see Tanaka F, 2008)
- Flogosis histopathological signs
- Titration of inflammation mediator expression in colon tissue extracts: TNFalfa, IL6, IL-1 beta, and Cox2 by RT-PCR.

### Statistical analysis of results

Data are expressed as mean ± SD. Group differences are analyzed by t-test p<0.05 being significant.

### Results

All the animals survived the treatment. While control animals (group 1) progressively gained weight during the week, DSS treated animals displayed weight loss beginning on fourth day (table 1). Weight loss was associated to liquid blood mixed faeces, with increase of colitis activity index (table 1). Both body weight loss and colitis activity index displayed to be remarkably lower in animals daily administered with MSC-MV intraperitoneal injections in addition to DSS. Water intake was meaningfully diminished in DSS administered animals, but was not meaningfully different for two experimental groups with or without MSC-MVs (table 1).
Table 1 shows body weight values, disease activity index and water intake on sixth day of experimentation for control animals without colitis induction administered only with PBS (vehicle/PBS), animals with DSS induced colitis and only PBS administered (DSS/PBS) and animals with DSS induced colitis and administered with injections of microvesicles isolated from mesenchymal stem cells and PBS suspended (MV/DSS).
Disease activity index (score Stool) has been evaluated according to following scale:
normal/semi-solid faeces = 1; semi-solid haematic faeces = 2; liquid haematic faeces = 3

**Table 1**

| EXPERIMENTAL GROUP | BODY WEIGHT (g) | STOOL SCORE | WATER INTAKE (mL) |
|---|---|---|---|
| | N=4 | N=5 | N=4 |
| (DAY 6) | Mean ± SD | Mean ± SD | Mean ± SD |
| Vehicle/PBS | 17.40 ± 0.57 | 1.0 ± 0 | 7.450 ± 3.977 |
| DSS/PBS | 15.60 ± 0.93 * | 2.5 ± 0 * | 3.160 ± 0.844* |
| DSS/MV | 17.15 ± 0.75 § | 1.9 ± 0.23 * § | 3.850 ± 0.904* |

| | | | |
|---|---|---|---|
| * Different from group vs Vehicle/PBS, p<0.05 § Different from group vs DSS/PBS, p<0.05 | | | |

Figure 10 shows RT-PCR analysis of TNFalfa, IL-1beta, IL-6 and Cox2 gene expression on colon extracted RNAs. DSS treatment results in remarkable increment of flogosis markers. Administration of MSC-MVs to DSS treated mice reduced meaningfully the activation of TNFalfa, IL-1 beta, while reduction of IL-6 and Cox2 displayed to be lower than significance limit. Histological analysis (Figure 11A and B) displayed inflammatory infiltrate both in group 2 and group 3. Although morphologic differences for two groups have not been detected, it is likely that such infiltrate is less active in group 3 than group 2, due to reduced expression of flogosis indices (Figure 10) together with lower clinical activity (table 1). In conclusion, these results show that MSC-MVs are suitable to reduce inflammatory reaction associated to experimental colitis resulting in reduction of disease activity index and amelioration of general conditions. MSC-MVs improve clinical picture and the flogistic response in an animal model of intestinal inflammatory disease.

### BIBLIOGRAPHY

- Augello A, Tasso R, Negrini SM, Amateis A, Indiveri F, Cancedda R, Pennesi G. Bone marrow mesenchymal progenitor cells inhibit lymphocyte proliferation by activation of the programmed death 1 pathway. Eur J Immunol 2005;35:1482-90.
- Bruno S, Grange C, Deregibus MC, Saviozzi S, Collino F, Bussolati B, Tetta C, Camussi G (2009) "Mesenchymal stem cell-derived microvesicles protect against acute tubular injury" J Am Soc Nephrol 20(5): 1053-1067
- Caimi PF, Reese J, Lee Z, Lazarus HM. Emerging therapeutic approaches for multipotent mesenchymal stromal cells. Curr Opin Hematol. 2010;17:505-13.
- Camussi G, Deregibus MC, Bruno S, Cantaluppi V, Biancone L.

Exosomes/microvesicles as a mechanism of cell-to-cell communication. Kidney lnt. 2010 78:838-48.
- Chan JL, Tang KC, Patel AP, Bonilla LM, Pierobon N, Ponzio NM, Rameshwar P. Antigen-presenting property of mesenchymal stem cells occurs during a narrow window at low levels of interferon-gamma. Blood. 2006 15;107:4817-24
- Comoli P, Ginevri F, Maccario R, Frassoni F, Locatelli F (2008) "Human mesenchymal stem cell inhibit antibody production induced in vitro by allostimulation" Nephrol Dial Transplant 23:1196-1202
- Corcione A, Benvenuto F, Ferretti E, Giunti D, Pistoia V, Uccelli A (2006) "Human mesenchymal stem cells modulate B-cells functions" Blood 107(1): 367-372
- Escudier B, Dorval T, Chaput N, André F, Caby MP, Novault S, Flament C, Leboulaire C, Borg C, Amigorena S, Boccaccio C, Bonnerot C, Dhellin O, Movassagh M, Piperno S, Robert C, Serra V, Valente N, Le Pecq JB, Spatz A, Lantz O, Tursz T, Angevin E, Zitvogel L. Vaccination of metastatic melanoma patients with autologous dendritic cell (DC) derivedexosomes: results of the first phase I clinical trial. J. Transl. Med. 3, 10 (2005).
- Gatti S, Bruno S, Deregibus MC, Sordi A, Cantaluppi V, Tetta C, Camussi G. Microvesicles derived from human adult mesenchymal stem cells protect against ischaemia-reperfusion-induced acute and chronic kidney injury. Nephrol Dial Transplant. 2011 May;26(5):1474-83. Epub 2011 Feb 15
- Herrera MB, Fonsato V, Gatti S, Deregibus MC, Sordi A, Cantarella D, Calogero R, Bussolati B, Tetta C, Camussi G. Human liver stem cell-derived microvesicles accelerate hepatic regeneration in hepatectomized rats. J Cell Mol Med. 2010;14:1605-18.
- Kawada M, Arihiro A, Mizoguchi E. Insights from advances in research of chemically induced experimental models of human inflammatory bowel disease. World J Gastroenterol. 2007(42):5581-93.
- Krampera M, Glennie S, Dyson J, Scott D, Simpson E, Dazzi F (2003) "Bone marrow mesenchymal stem cells inhibit the response of naïve and memory antigen-specific T cells to their cognate peptide" Blood 101: 3722-3729
- Lamparski HG, Metha-Damani A, Jenq-Yuan Y, Le Pecq JB (2002) "Production and characterization of clinical grade exosomes derived from dendritic cells" J Immunol Methods 270: 211-226
- Meirelles Lda S, Fontes AM, Covas DT, Caplan Al. Mechanisms involved in the therapeutic properties of mesenchymal stem cells. Cytokine Growth Factor Rev. 2009;20:419-27.
- Morse MA, Garst J, Osada T, Khan S, Hobeika A, Clay TM, Valente N, Shreeniwas R, Sutton MA, Delcayre A, Hsu DH, Le Pecq JB, Lyerly HK.. A phase I study of dexosome immunotherapy in patients with advanced non-small cell lung cancer. J. Transl. Med. 3, 9 (2005).
- Nauta AJ, Fibbe WE (2007) "Immunomodulating properties of mesenchymal stromal cells" Blood 110(10): 3499-3506
- Rasmussen I, Le Blank K, Sundberg B, Ringden O (2007) "Mesenchymal stem cells stimulate antibody secretion in human B cells" Scandinavian Journal of Immunology 65: 336-343
- Ren G, Su J, Zhang L, Zhao X, Ling W, L'huillie A, Zhang J, Lu Y, Roberts Al, Ji W, Zhang H, Rabson AB, Shi Y (2009) "Species variation in the mechanisms of mesenchymal stem cell-mediated immunosuppression" Stem Cells 2009 27(8):1954-62
- Tabera S, Perez-Simon J, Diez-Campelo M, Sanchez-Abarca LI, Bianco B, Lopez A, Benito A, Ocio E, Sanchez-Guijo FM, Canino C, San Miguel JF (2008) "The effect of mesenchymal stem cells on the viabilità, proliferation and differentiation of B-lymphocytes" Haematologica 93(9): 1301-1309
- Tanaka F, Tominaga K, Ochi M, Tanigawa T, Watanabe T, Fujiwara Y, Ohta K, Oshitani N, Higuchi K, Arakawa T. Exogenous administration of mesenchymal stem cells ameliorates dextran sulfate sodium-induced colitis via anti-inflammatory action in damaged tissue in rats. Life Sci. 2008 Dec 5;83(23-24):771-9.
- Théry C, Ostrowski M, Segura E. Membrane vesicles as conveyors of immune responses. Nat Rev Immunol. 2009;9:581-93.
- Tolar J, Nauta AJ, Osborn MJ, et al. Sarcoma derived from cultured mesenchymal stem cells. Stem Cells 2007; 25:371-379
- Traggiai E, Volpi S, Schena F, Gattorno M, Ferlito F, Moretta L, Martini A (2008) "Bone marrow-derived mesenchymal stem cells induce both polyclonal expansion and differentiation of B cells isolated from healthy donors and systemic lupus erythematosus patients" Stem Cells 26: 562-569
- Valadi H, Ekström K, Bossios A, Sjöstrand M, Lee JJ, Lötvall JO. Exosome-mediated transfer of mRNAs and microRNAs is a novel mechanism of genetic exchange between cells. Nat Cell Biol. 2007;9:654-9.

## Claims

1. Microvesicles, or exosomes, isolated from mesenchymal stem cells for use in immunosuppressive therapy.

2. Microvesicles isolated from mesenchymal stem cells for use according to claim 1, in the treatment of acute and chronic inflammatory diseases and autoimmune diseases.

3. Microvesicles isolated from mesenchymal stem cells for use according to claim 1, in the treatment and prevention of transplant rejection.

4. Microvesicles isolated from mesenchymal stem cells for use according to claim 1, in the treatment and prevention of graft-versus-host-disease.

5. Microvesicles isolated from mesenchymal stem cells for use according to claim 1, in order to inhibit immune reaction developing as result of allogeneic/xenogeneic cell transplant or gene therapy.

6. Pharmaceutical composition comprising or consisting of microvesicles isolated from mesenchymal stem cells, as active principle, optionally annexin bound, together with one or more pharmaceutically acceptable excipients and/or adjuvants, for use in immunosuppressive therapy.

7. Pharmaceutical composition for use according to claim 6, in the treatment of the acute and chronic inflammatory diseases and autoimmune diseases.

8. Pharmaceutical composition for use according to claim 6, in the treatment and prevention of transplant rejection.

9. Pharmaceutical composition for use according to claim 6, in the treatment and prevention of graft-versus-host-disease.

10. Pharmaceutical composition for use according to claim 6, in order to inhibit immune reaction developing as result of allogeneic/xenogeneic cell transplant or gene therapy.

## Patentansprüche

1. Mikrovesikel oder Exosomen, welche aus mesenchymalen Stammzellen isoliert sind, zur Verwendung in einer immunsuppresiven Therapie.

2. Mikrovesikel, welche aus mesenchymalen Stammzellen isoliert sind, zur Verwendung nach Anspruch 1, in der Behandlung von akuten und chronischen Entzündungserkrankungen und Autoimmunerkrankungen.

3. Mikrovesikel, welche aus mesenchymalen Stammzellen isoliert sind, zur Verwendung nach Anspruch 1, in der Behandlung und Vorbeugung einer Transplantatabstoßung.

4. Mikrovesikel, welche aus mesenchymalen Stammzellen isoliert sind, zur Verwendung nach Anspruch 1, in der Behandlung und Vorbeugung einer Graft-versus-Host-Reaktion.

5. Mikrovesikel, welche aus mesenchymalen Stammzellen isoliert sind, zur Verwendung nach Anspruch 1, zum Inhibieren einer Immunreaktion, die sich als Ergebnis eines allogenen/xenogenen Zelltransplantats oder Gentherapie entwickelt.

6. Pharmazeutische Zusammensetzung umfassend oder bestehend aus Mikrovesikeln, welche aus mesenchymalen Stammzellen isoliert sind, als Wirkstoff, gegebenenfalls Annexin-gebunden, zusammen mit einem oder mehreren pharmazeutisch wirksamen Hilfsstoffen und/oder Adjuvantien zur Verwendung in einer immunsuppressiven Therapie.

7. Pharmazeutische Zusammensetzung zur Verwendung nach Anspruch 6, in der Behandlung der akuten und chronischen Entzündungserkrankungen und Autoimmunerkrankungen.

8. Pharmazeutische Zusammensetzung zur Verwendung nach Anspruch 6, in der Behandlung und Vorbeugung einer Transplantatabstoßung.

9. Pharmazeutische Zusammensetzung zur Verwendung nach Anspruch 6, in der Behandlung und Vorbeugung einer Graft-versus-Host-Reaktion.

10. Pharmazeutische Zusammensetzung zur Verwendung nach Anspruch 6, zum Inhibieren einer Immunreaktion, die sich als Ergebnis eines allogenen/xenogenen Zelltransplantats oder Gentherapie entwickelt.

## Revendications

1. Microvésicules, ou exosomes, isolés de cellules souches mésenchymateuses à utiliser dans une thérapie immunosuppressive.

2. Microvésicules isolées provenant de cellules souches mésenchymateuses à utiliser selon la revendication 1, dans le traitement de maladies auto-immunes et de maladies inflammatoires aiguës et chroniques.

3. Microvésicules isolées provenant de cellules souches mésenchymateuses à utiliser selon la revendication 1, dans le traitement et la prévention du rejet de greffe.

4. Microvésicules isolées provenant de cellules souches mésenchymateuses à utiliser selon la revendication 1, dans le traitement et la prévention de la réaction de greffe contre hôte.

5. Microvésicules isolées de cellules souches mésenchymateuses à utiliser selon la revendication 1, afin d'inhiber le développement d'une réaction immunitaire en résultat d'un transplant cellulaire allogénique / xénogénique ou d'une thérapie génique.

6. Composition pharmaceutique comprenant des ou consistant en microvésicules isolées provenant de cellules souches mésenchymateuses, comme principe actif, de manière facultative liées à l'annexine, en même temps qu'un ou plusieurs excipients et/ou adjuvants acceptables d'un point de vue pharmaceutique, à utiliser dans une thérapie immunosuppressive.

7. Composition pharmaceutique à utiliser selon la revendication 6, dans le traitement de maladies auto-immunes et de maladies inflammatoires aiguës et chroniques.

8. Composition pharmaceutique à utiliser selon la revendication 6, dans le traitement et la prévention du rejet de greffe.

9. Composition pharmaceutique à utiliser selon la revendication 6, dans le traitement et la prévention de la réaction de greffe contre hôte.

10. Composition pharmaceutique à utiliser selon la revendication 6, afin d'inhiber le développement d'une réaction immunitaire en résultat d'un transplant cellulaire allogénique / xénogénique ou d'une thérapie génique.
